# EUROPEAN PATENT APPLICATION

(11) **EP 4 809 928 A1**
(43) Date of publication of application: **23.09.2026**
(21) Application number: 26164381.1
(22) Date of filing: 12.03.2026
(51) Int. Cl.: A61B 6/00, A61B 6/50, A61B 6/46

(54) **IMAGING CONTROL DEVICE, METHOD, AND PROGRAM, AND RADIOGRAPHY SYSTEM**

(30) Priority: 17.03.2025 JP 2025042896
(71) Applicant: FUJIFILM Corporation, Tokyo 106-8620 (JP)
(72) Inventor: TAKAHASHI, Tomoyuki, Kaisei-machi, Ashigarakami-gun, Kanagawa, 258-8538 (JP)
(74) Representative: Dehns Germany Partnerschaft mbB

(57) **Abstract**

A processor is configured to provide a first function of performing radiography and bone density measurement of a subject for a purpose of the bone density measurement, and a second function of performing the bone density measurement in an assistive manner for a purpose of general imaging of the subject, and switch between the first function and the second function.

## Description

### BACKGROUND

### Technical Field

The present disclosure relates to an imaging control device, method, and program, and a radiography system.

### Related Art

A dual X-ray absorptiometry (DXA) method is known as one of representative bone density measurement methods used for diagnosis of a bone disease such as osteoporosis. In addition, an X-ray imaging system capable of performing X-ray imaging using the DXA method and general imaging has also been proposed (see, for example, JP2023-183893A).

In the X-ray imaging system capable of performing the X-ray imaging using the DXA method and the general imaging, a function of measuring a bone density using the DXA method and a function of performing imaging for a different chief complaint other than osteoporosis or for a medical examination by the general imaging are switchable. On the other hand, it is considered to simply measure the bone density while performing the general imaging. For example, in a case where chest imaging is performed, the acquired X-ray image may include the lumbar vertebrae. In this case, the bone density can be simply measured by analyzing a bone region such as the lumbar vertebrae included in the X-ray image.

On the other hand, the functions required in the apparatus are different between the X-ray imaging using the DXA method and the general imaging. For example, in the X-ray imaging using the DXA method and the general imaging, a guide function for positioning, whether or not it is necessary to confirm an image after capturing, imaging conditions, and the like are different. Therefore, it is desirable to perform imaging by providing functions suitable for each of the X-ray imaging using the DXA method and the general imaging.

### SUMMARY OF THE INVENTION

The present disclosure has been made in view of the above circumstances, and an object of the present disclosure is to provide functions suitable for each of radiography and general imaging for the purpose of bone density measurement such as the DXA method.

An imaging control device according to the present disclosure comprises a processor, in which the processor is configured to provide a first function of performing radiography and bone density measurement of a subject for a purpose of the bone density measurement, and a second function of performing the bone density measurement in an assistive manner for a purpose of general imaging of the subject, and switch between the first function and the second function.

In the imaging control device according to the present disclosure, display of a guide for positioning at a time of imaging may be turned on in the first function, and the display of the guide may be turned off in the second function.

In the imaging control device according to the present disclosure, display of a confirmation image for confirming a measurement target bone for the bone density measurement may be turned on after imaging in the first function, and the display of the confirmation image may be turned off in the second function.

In the imaging control device according to the present disclosure, a radiation dose at a time of imaging in the first function may be smaller than a radiation dose at a time of imaging in the second function.

In the imaging control device according to the present disclosure, a function of deriving a pseudo image with a tube voltage different from a tube voltage at a time of imaging may be turned off in the first function, and the function of deriving the pseudo image may be turned on in the second function.

In the imaging control device according to the present disclosure, the processor may be configured to receive designation of whether to perform imaging for the bone density measurement or to perform the general imaging, and switch between the first function and the second function based on the designation.

In the imaging control device according to the present disclosure, the processor may be configured to switch between the first function and the second function based on an imaging condition.

In the imaging control device according to the present disclosure, the processor may be configured to switch between the first function and the second function according to whether imaging is performed using two stacked radiation detectors or the imaging is performed using one radiation detector in a radiography apparatus.

In the imaging control device according to the present disclosure, the processor may be configured to, after imaging, switch between the first function and the second function according to whether or not a measurement target bone for the bone density measurement is included in an acquired radiographic image.

An imaging control method according to the present disclosure comprises, via a computer, providing a first function of performing radiography and bone density measurement of a subject for a purpose of the bone density measurement, and a second function of performing the bone density measurement in an assistive manner for a purpose of general imaging of the subject, and switching between the first function and the second function.

An imaging control program according to the present disclosure causes a computer to execute a procedure for providing a first function of performing radiography and bone density measurement of a subject for a purpose of the bone density measurement, and a second function of performing the bone density measurement in an assistive manner for a purpose of general imaging of the subject and a procedure for switching between the first function and the second function.

The technology of the present disclosure may be applied to a program product.

A radiography system according to the present disclosure comprises a radiation source that emits radiation toward a subject, a plurality of radiation detectors that are stacked on each other, the plurality of radiation detectors acquiring a plurality of radiographic images having different energy distributions by simultaneously detecting radiation transmitted through the subject, and the imaging control device according to the present disclosure.

According to the present disclosure, it is possible to provide functions suitable for each of radiography and general imaging for the purpose of bone density measurement.

### BRIEF DESCRIPTION OF THE DRAWINGS

FIG. 1 is a schematic block diagram showing a configuration of a radiography system to which an imaging control device according to an embodiment of the present disclosure is applied.
FIG. 2 is a diagram showing a schematic configuration of the imaging control device according to the embodiment of the present disclosure.
FIG. 3 is a diagram showing a functional configuration of the imaging control device according to the embodiment of the present disclosure.
FIG. 4 is a diagram showing a setting screen of an imaging menu.
FIG. 5 is a diagram showing an operation screen displayed at the time of imaging.
FIG. 6 is a diagram showing an operation screen on which a confirmation image is displayed.
FIG. 7 is a diagram showing a pseudo image derivation screen.
FIG. 8 is a diagram showing the pseudo image derivation screen.
FIG. 9 is a flowchart showing processing performed in the present embodiment.

### DETAILED DESCRIPTION

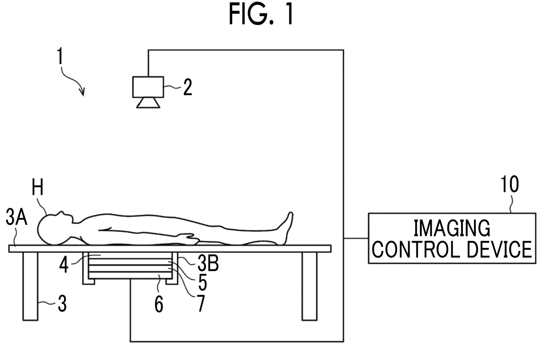

Hereinafter, an embodiment of the present disclosure will be described with reference to the drawings. FIG. 1 is a schematic block diagram showing a configuration of a radiography system to which an imaging control device according to the embodiment of the present disclosure is applied. As shown in FIG. 1, the radiography system according to the present embodiment comprises an imaging apparatus 1 and an imaging control device 10 according to the present embodiment.

The imaging apparatus 1 is an imaging apparatus that performs energy subtraction using a so-called one-shot method of irradiating a first radiation detector 5 and a second radiation detector 6 with radiation, such as X-rays, emitted from a radiation source 2 and transmitted through a subject H lying on an imaging table 3, at different energy levels, respectively. At the time of imaging, as shown in FIG. 1, a scattered ray removal grid (hereinafter, simply referred to as a grid) 4, the first radiation detector 5, a radiation energy conversion filter 7 made of a copper plate or the like, and the second radiation detector 6 are disposed in order from a side closest to the radiation source 2, and the radiation source 2 is driven. The first and second radiation detectors 5, 6 are closely attached to the radiation energy conversion filter 7. Note that the grid 4, the first radiation detector 5, the radiation energy conversion filter 7, and the second radiation detector 6 are attachably and detachably attached below a top plate 3A of the imaging table 3 with an attachment portion 3B.

As a result, in the first radiation detector 5, a first radiographic image G1 of the subject H by low-energy radiation also including so-called soft rays is acquired. In addition, in the second radiation detector 6, a second radiographic image G2 of the subject H by high-energy radiation from which the soft rays are removed is acquired. The first and second radiographic images G1, G2 are input to the imaging control device 10.

The first and second radiation detectors 5, 6 can perform recording and reading-out of the radiographic images repeatedly. A so-called direct-type radiation detector that directly receives emission of the radiation and generates a charge may be used, or a so-called indirect-type radiation detector that converts the radiation into visible light and then converts the visible light into a charge signal may be used. In addition, as a method for reading out a radiographic image signal, it is desirable to use a so-called thin film transistor (TFT) readout method in which the radiographic image signal is read out by turning a TFT switch on and off, or a so-called optical readout method in which the radiographic image signal is read out by emission of reading light, but other methods may also be used without being limited to these methods.

Note that in the imaging apparatus 1, only one radiation detector may be attached to the attachment portion 3B to perform imaging of the subject H.

The grid 4 is configured by lead that does not transmit the radiation and an interspace material, such as aluminum or fiber that easily transmits the radiation, which are disposed alternately with a fine grid density of, for example, about 4.0 lines/mm. By using the grid 4, a scattered ray component of the radiation transmitted through the subject H can be removed, but cannot be completely removed. Therefore, the first and second radiographic images G1, G2 also include a primary ray component of the radiation transmitted through the subject H as well as the scattered ray component.

The primary ray component is a signal component having a pixel value represented by the radiation that reaches the radiation detector without being scattered by the subject H in the radiation that is transmitted through the subject H. On the other hand, the scattered ray component is a signal component having a pixel value represented by the radiation that reaches the radiation detector by being scattered by the subject H in the radiation that is transmitted through the subject H.

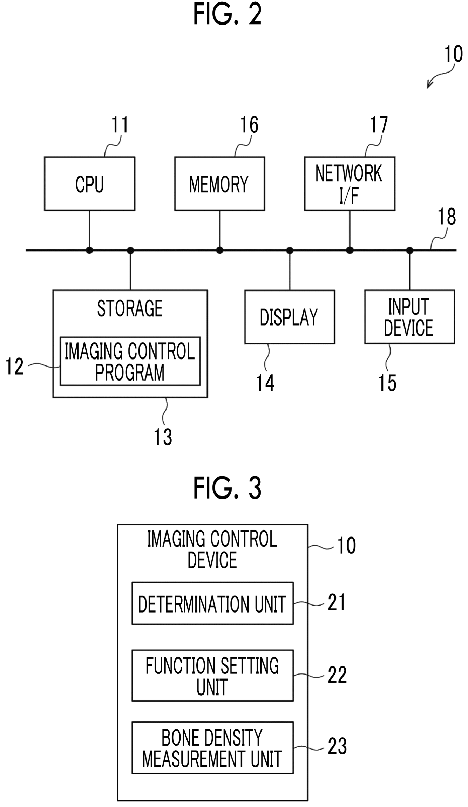

Next, the imaging control device according to the present embodiment will be described. First, a hardware configuration of the imaging control device according to the present embodiment will be described with reference to FIG. 2. As shown in FIG. 2, the imaging control device 10 is a computer, such as a workstation, a server computer, and a personal computer, and comprises a central processing unit (CPU) 11, a non-volatile storage 13, and a memory 16 as a temporary storage area. In addition, the imaging control device 10 comprises a display 14, such as a liquid crystal display, an input device 15, such as a keyboard and a mouse, and a network interface (I/F) 17 connected to a network (not shown). The CPU 11, the storage 13, the display 14, the input device 15, the memory 16, and the network I/F 17 are connected to a bus 18. Note that the CPU 11 is an example of a processor according to the present disclosure.

The storage 13 is realized by a hard disk drive (HDD), a solid state drive (SSD), a flash memory, or the like. An imaging control program 12 installed in the imaging control device 10 is stored in the storage 13 serving as a storage medium. The CPU 11 reads out the imaging control program 12 from the storage 13, loads the imaging control program 12 into the memory 16, and executes the loaded imaging control program 12.

Note that the imaging control program 12 is stored in a storage device of the server computer connected to the network or in a network storage in a state of being accessible from the outside, and is downloaded and installed on the computer that constitutes the imaging control device 10 in response to the request. Alternatively, the imaging control program 12 is distributed by being recorded on a recording medium, such as a digital versatile disc (DVD) or a compact disc read only memory (CD-ROM), and is installed on the computer that constitutes the imaging control device 10 from the recording medium.

Next, a functional configuration of the imaging control device according to the present embodiment will be described. FIG. 3 is a diagram showing the functional configuration of the imaging control device according to the present embodiment. As shown in FIG. 3, the imaging control device 10 comprises a determination unit 21, a function setting unit 22, and a bone density measurement unit 23. Then, the CPU 11 executes the imaging control program 12 to function as the determination unit 21, the function setting unit 22, and the bone density measurement unit 23. Note that the bone density measurement unit 23 may be provided as a separate device from the imaging control device 10 in the present embodiment.

The imaging control device 10 according to the present embodiment switches between a first function of performing radiography of the subject using the DXA method for the purpose of bone density measurement and a second function of measuring bone density for the purpose of the general imaging while performing the general imaging in the imaging apparatus 1.

First, the bone density measurement unit 23 will be described. In both the first function and the second function, the bone density measurement is performed using, for example, the method described in JP2023-082549A and the like. Hereinafter, the method described in JP2023-082549A will be described.

First, the bone density measurement unit 23 performs imaging of the subject H by the imaging apparatus 1 and acquires the first radiographic image G1 and the second radiographic image G2 of the subject H from the first and second radiation detectors 5, 6. The scattered ray components may be removed from the acquired first radiographic image G1 and second radiographic image G2. The bone density measurement unit 23 derives a bone part image Gb in which only the bone part of the subject H included in the first radiographic image G1 and the second radiographic image G2 is extracted by performing weighting subtraction between the corresponding pixels, on the first radiographic image G1 and the second radiographic image G2, as shown in Expression (1). (x, y) represents a pixel position of the first radiographic image G1 and the second radiographic image G2.$Gb \left(x, y\right) = G 1 \left(x, y\right) - α 1 \times G 2 \left(x, y\right)$

Then, the bone density measurement unit 23 derives a bone density B(x, y) corresponding to each pixel by converting each pixel value Gb(x, y) of the bone region of the bone part image Gb into a pixel value of the bone part image in a case where the pixel value Gb(x, y) is acquired under a reference imaging condition. In this case, in consideration of the fact that the larger body thickness of the subject H and higher tube voltage, the lower contrast of the bone part image, the bone density measurement unit 23 acquires the correction coefficient with reference to a look-up table that defines a relationship between the body thickness and the correction coefficient prepared for each tube voltage, and derives the bone density by correcting the pixel value of each pixel of the bone part image Gb by the acquired correction coefficient.

Note that in a case of the second function, the bone density may be derived from the pixel value of the bone part included in any one of the first radiographic image G1 or the second radiographic image G2, instead of the method described in JP2023-082549A. In this case, the bone density may be derived using a derivation model in which machine learning is performed so as to derive the bone density from the pixel value of the bone part.

Note that in a case where the purpose is the bone density measurement, the imaging part of the subject H is the proximal femur or the lumbar vertebrae. In a case where the purpose is the general imaging, imaging of the imaging part such as the chest and the abdomen designated by the doctor is performed.

In a case where the radiography is performed using the DXA method for the purpose of the bone density measurement, the proximal femur or the lumbar vertebrae is the imaging part, and thus it is necessary to appropriately position the imaging part. Therefore, it is necessary to perform guide display for positioning so that the operator can appropriately perform positioning of the imaging part. On the other hand, in a case of the general imaging, the imaging part is designated as the chest, the abdomen, or the limbs, and thus, strict positioning is not required as compared with the case of performing the bone density measurement.

In addition, in a case where the radiography is performed using the DXA method, it is necessary to confirm whether the imaging part such as the proximal femur or the lumbar vertebrae is appropriately included in the acquired image after imaging. On the other hand, in a case of the general imaging, the imaging part is designated as the chest, the abdomen, or the limbs, and thus, strict confirmation is not necessary.

In addition, in a case where the radiography is performed using the DXA method, it is sufficient to know the pixel value. Therefore, it is not necessary to acquire a high-quality image in which detailed interpretation of a lesion or the like can be performed, as with the general imaging. As a result, in a case where the purpose is the bone density measurement, the size of the image may be smaller than that of the general imaging. Smaller image size is less susceptible to the high-frequency noise. Therefore, the radiation dose at the time of imaging may be lower than that in the general imaging.

In addition, for the image acquired by the general imaging, an X-ray image having a contrast acquired at a tube voltage different from the tube voltage at the time of imaging may be pseudo-generated. On the other hand, in a case where the bone density measurement is performed, such an image having a different contrast is not generated.

The imaging control device 10 according to the present embodiment switches between the first function of performing the radiography and the bone density measurement of the subject using the DXA method for the purpose of the bone density measurement, and the second function of performing the general imaging of the subject and the bone density measurement by performing the bone density measurement in an assistive manner for the purpose of the general imaging of the subject.

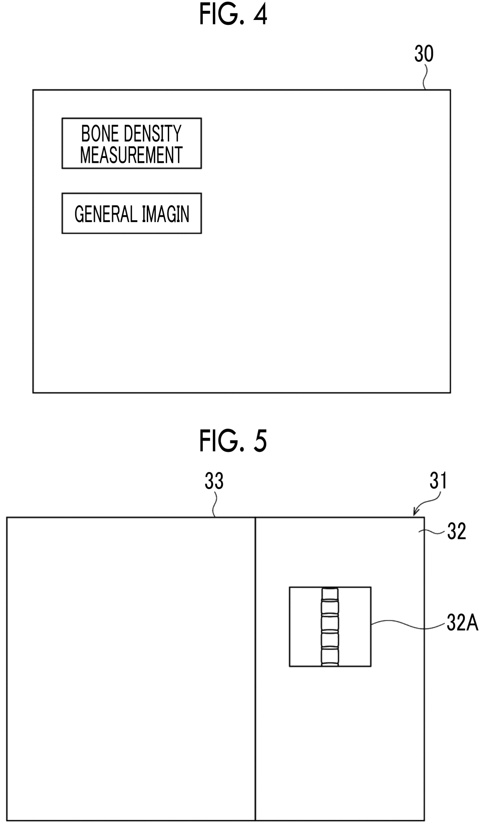

The determination unit 21 of the imaging control device 10 determines whether the imaging of the subject H is the radiography using the DXA method or the general imaging. In the imaging control device 10 according to the present embodiment, an imaging menu can be set at the time of imaging. In a case of setting the imaging menu, as shown in FIG. 4, a selection screen 30 is displayed on the display 14, allowing the operator to select whether the purpose of the imaging is the radiography using the DXA method for the bone density measurement or the general imaging. The determination unit 21 determines whether to perform the radiography using the DXA method or to perform the general imaging, based on the selection of the operator.

In addition, as described above, the radiography using the DXA method is performed with a lower dose than the general imaging. Therefore, in a case where the dose of the radiation included in the set imaging condition is smaller than a predetermined threshold value, the determination unit 21 may determine that the imaging is the radiography using the DXA method, and in other cases, the determination unit 21 may determine that the imaging is the general imaging.

The function setting unit 22 switches between the first function of performing the radiography and the bone density measurement of the subject using the DXA method for the purpose of the bone density measurement, and the second function of performing the general imaging of the subject and the bone density measurement by performing the bone density measurement in an assistive manner for the purpose of the general imaging of the subject, based on the determination result of the determination unit 21.

In a case where the determination unit 21 determines that the imaging is the radiography using the DXA method, the function setting unit 22 turns on the display of the guide for positioning at the time of imaging and displays a guide image on the display 14. FIG. 5 is a diagram showing an operation screen displayed at the time of imaging. As shown in FIG. 5, an operation screen 31 has a guide region 32 and a display region 33 for displaying the X-ray image. A guide image 32A is displayed in the guide region 32. The guide image 32A represents the lumbar vertebrae to be imaged for the bone density measurement. The operator performs positioning of the subject H with reference to the guide image 32A. Displaying the guide image is an example of the first function of the present disclosure.

On the other hand, in a case where the determination unit 21 determines that the imaging is the general imaging, the function setting unit 22 turns off the display of the guide for the positioning at the time of imaging and does not display the guide image 32A. Not displaying the guide image 32A is an example of the second function of the present disclosure.

In addition, in a case where the determination unit 21 determines that the imaging is the radiography using the DXA method, the function setting unit 22 sets the dose included in the imaging condition to a low dose for the bone density measurement. On the other hand, in a case where it is determined that the imaging is the general imaging, the function setting unit 22 sets the dose according to the imaging part. Setting the low dose for the bone density measurement is an example of the first function of the present disclosure. Setting the dose according to the imaging part is an example of the second function of the present disclosure. Note that in this case, the imaging condition does not need to be set by the operator.

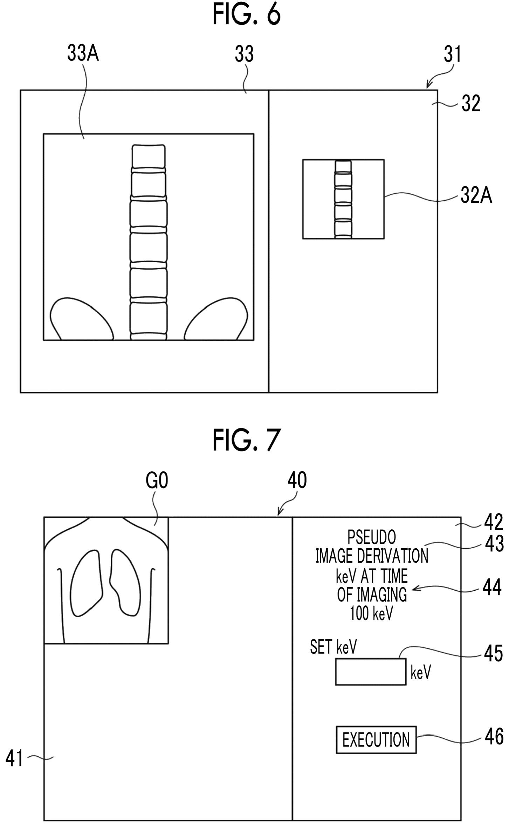

In addition, in a case where the determination unit 21 determines that the imaging is the radiography using the DXA method, the function setting unit 22 turns on the display of a confirmation image for confirming the measurement target bone and displays the confirmation image after imaging. FIG. 6 is a diagram showing the operation screen on which the confirmation image is displayed. As shown in FIG. 6, the radiographic image acquired by imaging is displayed as a confirmation image 33A in the display region 33 of the operation screen 31. The radiographic image to be displayed may be any of the first radiographic image G1 or the second radiographic image G2. The operator can compare the displayed confirmation image 33A with the guide image 32A and confirm whether or not the imaging is performed by appropriate positioning. Displaying the confirmation image 33A is an example of the first function of the present disclosure.

On the other hand, in a case where the determination unit 21 determines that the imaging is the general imaging, the function setting unit 22 turns off the display of the confirmation image for confirming the measurement target bone and does not display the confirmation image 33A. Not displaying the confirmation image 33A is an example of the second function of the present disclosure.

In addition, in a case where the determination unit 21 determines that the imaging is the general imaging, the function setting unit 22 may turn on a function of deriving a pseudo image as though the imaging is performed at a tube voltage different from the tube voltage at the time of imaging, and may display a pseudo image derivation screen for executing processing of deriving the pseudo image on the display 14. Turning on the function of deriving the pseudo image and displaying the pseudo image derivation screen is an example of the second function of the present disclosure. Turning off the function of deriving the pseudo image and not displaying the pseudo image derivation screen is an example of the first function of the present disclosure.

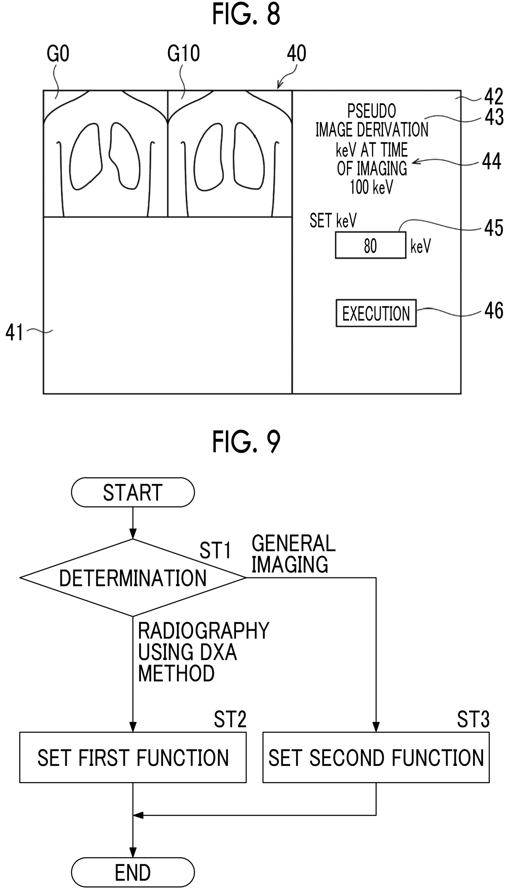

FIG. 7 is a diagram showing the pseudo image derivation screen. As shown in FIG. 7, on a pseudo image derivation screen 40, a display region 41 for a radiographic image G0 acquired by the imaging and the pseudo image, and a setting region 42 are displayed. In the setting region 42, a text 43 of "pseudo image derivation", a tube voltage 44 at the time of imaging (here, 100 keV), an input box 45 for the tube voltage for deriving the pseudo image, and an execution button 46 are displayed. The operator inputs the tube voltage for deriving the pseudo image to the input box 45 and presses the execution button 46, and as a result, the function setting unit 22 derives the pseudo image at the set tube voltage. The pseudo image G10 is displayed in the display region 41 in a state of being arranged side by side with the acquired radiographic image G0, as shown in FIG. 8.

Hereinafter, processing performed in the present embodiment will be described. FIG. 9 is a flowchart showing the processing performed in the present embodiment. First, the determination unit 21 determines whether the imaging of the subject H is the radiography using the DXA method or the general imaging (step ST1). In a case where it is determined that the imaging is the radiography using the DXA method, the function setting unit 22 sets the first function (step ST2), and ends the processing. In a case where it is determined that the imaging is the general imaging, the function setting unit 22 sets the second function (step ST3), and ends the processing.

As described above, in the imaging control device 10 according to the present embodiment, the first function of performing the radiography and the bone density measurement of the subject using the DXA method for the purpose of the bone density measurement, and the second function of performing the general imaging of the subject and the bone density measurement by performing the bone density measurement in an assistive manner for the purpose of the general imaging of the subject are provided, and the first function and the second function are switched. Therefore, it is possible to provide functions suitable for each of the radiography and the general imaging for the purpose of the bone density measurement such as the DXA method.

Specifically, it is possible to switch and provide functions of displaying the guide for positioning according to the purpose, displaying the confirmation image, setting the imaging condition, and deriving the pseudo image. In particular, in a case where the general imaging is performed, it is not necessary to display the guide for imaging and the confirmation image, and displaying the guide for positioning or the confirmation image is troublesome for the operator. Therefore, as in the present embodiment, by switching between the first function and the second function, it is possible to display a screen that is easy for the operator to operate according to the purpose of imaging.

Note that in the above-described embodiment, in a case where the radiography is performed using the DXA method, the first and second radiation detectors 5, 6 are used. However, in the general imaging, only any one of the first or second radiation detectors 5, 6 may be used. Therefore, in the imaging apparatus 1, the first function and the second function may be switched depending on whether both of the first and second radiation detectors 5, 6 are used or any one of the first or second radiation detectors 5, 6 is used.

In addition, in the above-described embodiment, in a case where the radiography is performed using the DXA method, the radiographic image includes the proximal femur or the lumbar vertebrae, which is a measurement target bone. Therefore, in a case where the measurement target bone is included in the X-ray image acquired by the imaging, the imaging control device may switch to the first function, and in other cases, the imaging control device may switch to the second function. In this case, the first function and the second function provide functions after imaging (for example, the display of the confirmation image and the display of the pseudo image derivation screen).

In addition, in the above-described embodiment, the first and second radiographic images G1, G2 are acquired by the one-shot method, but the present disclosure is not limited thereto. The first and second radiographic images G1, G2 may be acquired by a so-called two-shot method in which the imaging is performed twice using only one radiation detector. In a case of the two-shot method, a position of the subject H included in the first radiographic image G1 and the second radiographic image G2 may shift due to a body movement of the subject H. Therefore, in the first radiographic image G1 and the second radiographic image G2, it is preferable to perform the processing of the present embodiment after the registration of the subject is performed.

In addition, in the above-described embodiment, the present embodiment is applied to the system that captures the first and second radiographic images G1, G2 of the subject H using the first and second radiation detectors 5, 6, but the present disclosure is not limited thereto. It goes without saying that the technology of the present disclosure can be applied even in a case where the first and second radiographic images G1, G2 are acquired using an accumulative phosphor sheet instead of the radiation detector. In this case, the first and second radiographic images G1, G2 may be acquired by stacking two accumulative phosphor sheets, emitting radiation transmitted through the subject H, accumulating and recording radiographic image information of the subject H in each of the accumulative phosphor sheets, and photoelectrically reading the radiographic image information from each of the accumulative phosphor sheets. Note that the two-shot method may also be used in a case where the first and second radiographic images G1, G2 are acquired using the accumulative phosphor sheet.

In addition, the radiation in the embodiment described above is not particularly limited, and α-rays or γ-rays can be used in addition to X-rays.

In the present embodiment, each processing is executed by any computer. Moreover, any computer may execute these types of processing by a processor as hardware, a program as software, or a combination thereof. In this case, the processor is configured to execute various types of processing in the present embodiment in cooperation with the program, and can function as each unit or each means in the present embodiment. Further, the execution order of the processing by the processor is not limited to the above-described order and may be changed as appropriate. Any computer may be a general-purpose computer, a computer for a specific application, a workstation, or another system capable of executing each processing.

The processor may be configured by one or a plurality of types of hardware, and the type of hardware is not limited. For example, the processor may be configured by hardware, such as a central processing unit (CPU), a micro processing unit (MPU), a programmable logic device, such as a field programmable gate array (FPGA), a dedicated circuit for executing specific processing, such as an application specific integrated circuit (ASIC), a graphic processing unit (GPU), or a neural processing unit (NPU). In addition, the type of hardware may be a combination of different types of hardware. In a case where the plurality of types of hardware are configured to execute one or a plurality of types of processing of a certain processor, the plurality of types of hardware may exist in devices physically separated from each other or may exist in the same device. Additionally, in any embodiment, the order of each processing by the processor is not limited to the order described above and may be changed as appropriate. Note that the hardware is configured by an electrical circuit (circuitry) in which circuit elements, such as semiconductor elements, are combined, or the like.

Further, the program may be software, such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, storage media, other storages, or the like). The program may be divided and stored in a plurality of non-transitory computer-readable media that exist in devices physically separated from each other. The program code or the code segments may represent any combination of procedures, functions, subprograms, routines, subroutines, modules, software packages, classes, instructions, data structures, or program statements. The program code or the code segments may be connected to other code segments or hardware circuits by transmitting and receiving information, data, an argument, a parameter, or content of a memory.

Additionally, in the above-described embodiment, an aspect has been described in which the imaging control program 12 is stored (installed) in advance in the storage 13, but the present disclosure is not limited to this aspect. The imaging control program 12 may be provided in a form recorded on a recording medium, such as a compact disc read-only memory (CD-ROM), a digital versatile disc read-only memory (DVD-ROM), and a universal serial bus (USB) memory. Furthermore, the imaging control program 12 may be downloaded from an external device via a network.

The technology of the present disclosure extends to all types of program products. The program product includes any aspects of products for providing a program. For example, the program product includes a program provided through a network such as the Internet, a non-transitory computer-readable recording medium, such as a CD-ROM, a DVD, and a USB memory in which the program is stored, and the like.

Hereinafter, supplementary notes of the present disclosure will be described.

### Supplementary Note 1

An imaging control device comprising:
a processor,
wherein the processor is configured to:
   provide a first function of performing radiography and bone density measurement of a subject for a purpose of the bone density measurement, and a second function of performing the bone density measurement in an assistive manner for a purpose of general imaging of the subject; and
   switch between the first function and the second function.

### Supplementary Note 2

The imaging control device according to supplementary note 1,
wherein display of a guide for positioning at a time of imaging is turned on in the first function, and the display of the guide is turned off in the second function.

### Supplementary Note 3

The imaging control device according to supplementary note 1 or 2,
wherein display of a confirmation image for confirming a measurement target bone for the bone density measurement is turned on after imaging in the first function, and the display of the confirmation image is turned off in the second function.

### Supplementary Note 4

The imaging control device according to any one of supplementary notes 1 to 3,
wherein a radiation dose at a time of imaging in the first function is smaller than a radiation dose at a time of imaging in the second function.

### Supplementary Note 5

The imaging control device according to any one of supplementary notes 1 to 4,
wherein a function of deriving a pseudo image with a tube voltage different from a tube voltage at a time of imaging is turned off in the first function, and the function of deriving the pseudo image is turned on in the second function.

### Supplementary Note 6

The imaging control device according to any one of supplementary notes 1 to 5,
wherein the processor is configured to:
receive designation of whether to perform imaging for the bone density measurement or to perform the general imaging; and
switch between the first function and the second function based on the designation.

### Supplementary Note 7

The imaging control device according to any one of supplementary notes 1 to 5,
wherein the processor is configured to switch between the first function and the second function based on an imaging condition.

### Supplementary Note 8

The imaging control device according to any one of supplementary notes 1 to 5,
wherein the processor is configured to switch between the first function and the second function according to whether imaging is performed using two stacked radiation detectors or the imaging is performed using one radiation detector in a radiography apparatus.

### Supplementary Note 9

The imaging control device according to any one of supplementary notes 1 to 5,
wherein the processor is configured to, after imaging, switch between the first function and the second function according to whether or not a measurement target bone for the bone density measurement is included in an acquired radiographic image.

### Supplementary Note 10

An imaging control method comprising:
via a computer,
providing a first function of performing radiography and bone density measurement of a subject for a purpose of the bone density measurement, and a second function of performing the bone density measurement in an assistive manner for a purpose of general imaging of the subject; and
switching between the first function and the second function.

### Supplementary Note 11

An imaging control program causing a computer to execute:
a procedure for providing a first function of performing radiography and bone density measurement of a subject for a purpose of the bone density measurement, and a second function of performing the bone density measurement in an assistive manner for a purpose of general imaging of the subject; and
a procedure for switching between the first function and the second function.

### Supplementary Note 12

A radiography system comprising:
a radiation source that emits radiation toward a subject;
a plurality of radiation detectors that are stacked on each other, the plurality of radiation detectors acquiring a plurality of radiographic images having different energy distributions by simultaneously detecting radiation transmitted through the subject; and
the imaging control device according to any one of supplementary notes 1 to 9.

### Explanation of References

1: imaging apparatus
2: radiation source
3: imaging table
3A: top plate
3B: attachment portion
4: scattered ray removal grid
5, 6: radiation detector
7: radiation energy conversion filter
10: imaging control device
11: CPU
12: imaging control program
13: storage
14: display
15: input device
16: memory
17: network I/F
18: bus
21: determination unit
22: function setting unit
23: bone density measurement unit
30: selection screen
31: operation screen
32: guide region
32A: guide image
33: display region
33A: confirmation image
40: pseudo image derivation screen
41: display region
42: setting region
43: text
44: tube voltage during imaging
45: input box
46: execution button
Gb: X-ray image

## Claims

1. An imaging control device comprising:
a processor,
wherein the processor is configured to:
provide a first function of performing radiography and bone density measurement of a subject for a purpose of the bone density measurement, and a second function of performing the bone density measurement in an assistive manner for a purpose of general imaging of the subject; and
switch between the first function and the second function.

2. The imaging control device according to claim 1,
wherein display of a guide for positioning at a time of imaging is turned on in the first function, and the display of the guide is turned off in the second function.

3. The imaging control device according to claim 1 or 2,
wherein display of a confirmation image for confirming a measurement target bone for the bone density measurement is turned on after imaging in the first function, and the display of the confirmation image is turned off in the second function.

4. The imaging control device according to any one of claims 1 to 3,
wherein a radiation dose at a time of imaging in the first function is smaller than a radiation dose at a time of imaging in the second function.

5. The imaging control device according to any one of claims 1 to 4,
wherein a function of deriving a pseudo image with a tube voltage different from a tube voltage at a time of imaging is turned off in the first function, and the function of deriving the pseudo image is turned on in the second function.

6. The imaging control device according to any one of claims 1 to 5,
wherein the processor is configured to:
receive designation of whether to perform imaging for the bone density measurement or to perform the general imaging; and
switch between the first function and the second function based on the designation.

7. The imaging control device according to any one of claims 1 to 5,
wherein the processor is configured to switch between the first function and the second function based on an imaging condition.

8. The imaging control device according to any one of claims 1 to 5,
wherein the processor is configured to switch between the first function and the second function according to whether imaging is performed using two stacked radiation detectors or the imaging is performed using one radiation detector in a radiography apparatus.

9. The imaging control device according to any one of claims 1 to 5,
wherein the processor is configured to, after imaging, switch between the first function and the second function according to whether or not a measurement target bone for the bone density measurement is included in an acquired radiographic image.

10. An imaging control method comprising:
via a computer,
providing a first function of performing radiography and bone density measurement of a subject for a purpose of the bone density measurement, and a second function of performing the bone density measurement in an assistive manner for a purpose of general imaging of the subject; and
switching between the first function and the second function.

11. An imaging control program causing a computer to execute:
a procedure for providing a first function of performing radiography and bone density measurement of a subject for a purpose of the bone density measurement, and a second function of performing the bone density measurement in an assistive manner for a purpose of general imaging of the subject; and
a procedure for switching between the first function and the second function.

12. A radiography system comprising:
a radiation source that emits radiation toward a subject;
a plurality of radiation detectors that are stacked on each other, the plurality of radiation detectors acquiring a plurality of radiographic images having different energy distributions by simultaneously detecting radiation transmitted through the subject; and
the imaging control device according to any one of claims 1 to 9.
